# EUROPÄISCHE PATENTANMELDUNG

(11) **EP 2 286 724 A2**
(43) Veröffentlichungstag der Anmeldung: **23.02.2011**
(21) Anmeldenummer: 10005934.4
(22) Anmeldetag: 09.06.2010
(51) Int. Cl.: A61B 5/107

(54) **Vorrichtung zur Messung der Grösse einer intrakardialen Öffnung**

(30) Priorität: 21.08.2009 DE 102009038500
(71) Anmelder: Osypka, Peter, 79618 Rheinfelden-Herten (DE)
(72) Erfinder: Osypka, Peter, 79618 Rheinfelden-Herten (DE)
(74) Vertreter: Börjes-Pestalozza, Heinrich

(57) **Zusammenfassung**

Bei einer Vorrichtung (1) zur Messung des Durchmessers einer intrakardialen Öffnung (2) ist ein aus einem Metalldrahtgeflecht bestehender Hohlkörper (3) vorgesehen, welcher verdrillte Einzelstäbe aufweist, die den Mantel der durchbrochenen Wandung (5) des Hohlkörpers (3) bilden und welche eine Längsstabilisierung zwischen zwei die Stirnseite des Hohlkörpers (3) bildenden, elastisch verformbaren Metalldrahtgeflechten (6) bilden und in Gebrauchsstellung durch den Rand (17) der Öffnung (2) in einem Berührbereich in das Innere des Hohlkörpers (3) verformbar sind. Mit der Vorrichtung (1) ist somit die Größe der Öffnung (2) bestimmbar, indem in einem Röntgen- oder Ultraschallbild die Verformung der Einzelstäbe 4 durch die Öffnung (2) mit an dem Hohlkörper (3) und/oder an der Vorrichtung (1) dem Hohlkörper (3) benachbart angeordneten Markierungen (19, 20) vergleichbar ist.

## Beschreibung

Die Erfindung betrifft eine Vorrichtung zur Messung der Größe einer intrakardialen Öffnung, mit einem eine nachgiebige Wandung aufweisenden, in die Öffnung einführbaren und quer zur Einführungsrichtung expandierbaren Hohlkörper und mit wenigstens zwei strahlenundurchlässigen, in einem vorbestimmten Abstand zueinander angeordneten Markierungen, deren Abstand mit der Einformung des Hohlkörpers durch den Rand der zu messenden Öffnung verglichen werden kann, wobei der Hohlkörper flüssigkeitsdurchlässig und als ein Metallkäfig ausgebildet ist.

Eine vergleichbare Vorrichtung ist aus der US 2006/0173300 A1 bekannt. Der den Hohlkörper bildende Metallkäfig soll dabei in einem mittleren Bereich durch die Ränder der abzumessenden Öffnung eingedrückt werden, so dass daraus Rückschlüsse auf die Größe der Öffnung möglich sind. Der weitgehend einheitlich aufgebaute Metallkäfig soll also nur im Bereich der Lochungsränder eingedrückt werden, im Übrigen aber stabil bleiben, was bei üblichen derartigen Metallkäfigen in der Praxis kaum erreichbar ist. Vielmehr ist damit zu rechnen, dass der mittlere Bereich dieses Metallkäfigs insgesamt nach innen verformt wird und keine klare und deutliche Einschnürung im Bereich der Ränder einer abzumessenden Lochung auftritt.

Eine derartige Vorrichtung ist aus der DE 699 35 601 T2 bekannt, bei welcher ein an dem distalen Ende eines Katheders befestigter Ballon in die zu vermessende Öffnung gebracht und mit einem Kontrastmittel befüllt wird, bis ein Druckschwellwert erreicht ist oder an der Öffnung kein Links-Rechts-Shunt mehr beobachtet werden kann, wobei zur Beobachtung des Links-Rechts-Shunts Doppler-Echokardiographie verwendet wird. Die Größe der Öffnung kann bestimmt werden, indem der Ballon außerhalb des Patientenkörpers erneut mit derselben Menge des zugeführten Kontrastmittels befüllt und in verschiedene Öffnungen einer Matrize positioniert wird. Ferner können an dem Schaft des Katheters strahlenundurchlässige Markierungen angebracht sein.

Es hat sich herausgestellt, dass die Verformung des Ballons durch die Ränder der untersuchten Öffnung im Herzen im Röntgenbild relativ schwer erkennbar ist, sodass die Überwachung mit Doppler-Echokardiographie erforderlich ist.

Aus der DE 10 2007 018 763 U1 ist ein Ballonkatheder zum Ermitteln der Größe einer Öffnung bekannt, bei welchem mit Hilfe eines Betätigungselements die Aufweitbewegung des Ballons in eine in Erstreckungsrichtung des Schafts des Katheters verlaufende Verstellbewegung eines außerhalb des Patientenkörpers angeordneten, den Schaft ringförmig umgreifenden Anzeigeelements umwandelbar ist. Die Aufweitung des Ballons geschieht durch Einführung einer Flüssigkeit oder durch Aufblasen.

Es besteht deshalb die Aufgabe, eine Vorrichtung zur Messung der Größe einer intrakardialen Öffnung zu schaffen, bei welcher der Vorteil erhalten bleibt, kein Kontrastmittel zu benötigen, und bei welcher die Verwendung eines Ballons vermieden und eine einfache Handhabung erreicht werden kann, wobei die Verformung des Hohlkörpers im Röntgen- oder Ultraschallbild gut erkennbar und durch Vergleich mit den Markierungen zuverlässig bestimmbar ist, ohne dass der Hohlkörper selbst an unerwünschten Stellen verformt und die Größenbestimmung der Öffnung ungenau wird.

Erfindungsgemäß ist zur Lösung dieser Aufgabe bei einer Vorrichtung der eingangs genannten Art vorgesehen, dass der Hohlkörper in seinem mittleren Bereich zwischen seinen Stirnseiten unterbrochen und die Unterbrechung durch Einzelstäbe überbrückt ist, welche jeweils durch wenigstens zwei miteinander verdrillte Drähte gebildet sind.

Die verdrillten Drähte oder Drahtabschnitte stellen eine insbesondere längsgerichtete Stabilisierung des flüssigkeitsdurchlässigen Hohlkörpers bereit, die im Bereich der abzumessenden Öffnung genügend weich und nachgiebig ist, damit die Abmessung des Randes der Öffnung anhand der Verformung dieser verdrillten Drähte und Drahtabschnitte gut im Röntgen- oder Ultraschallbild erkannt werden kann. Gleichzeitig können die metallischen Stirnseiten den Hohlkörper weitgehend stabilisieren.

Dadurch, dass der Hohlkörper flüssigkeitsdurchlässig ausgebildet ist, wird der Patient möglichst wenig durch den in Gebrauchsstellung in der Öffnung angeordneten, die Öffnung nicht verschließende Hohlkörper beeinträchtigt, und der ballonlose Hohlkörper kann nach Gebrauch zum Entfernen leicht und platzsparend wieder zusammengefaltet werden, ohne dass hierbei ein Ballon stören würde.

Begünstigt wird die Handhabung und Bestimmung der Größe der Öffnung, wenn in Ausgangsstellung die Einzelstäbe parallel zueinander und zu einer Längsmittelachse des Hohlkörpers verlaufen. Die Einzelstäbe können also auf einer gedachten Mantelfläche eines zylindrischen Bereichs des Hohlkörpers angeordnet sein, so dass eine gut erkennbare Einbuchtung in Gebrauchsstellung im Bereich einer abzumessenden Öffnung entsteht.

Die Größe der Öffnung kann somit durch Vergleich der Größe der Einformung des Metallkäfigs durch die Ränder der Öffnung mit dem Abstand der Markierungen in einem Röntgen- oder Ultraschallbild ermittelt werden. Hierbei kann der Hohlkörper in der Öffnung kontrolliert so weit aufgeweitet oder expandiert werden, bis die Öffnung ausgefüllt ist.

Es hat sich aber herausgestellt, dass der Hohlkörper auch unkontrolliert expandiert werden kann, da die Verformung des Hohlkörpers durch die Öffnung im Röntgen- oder Ultraschallbild gut erkennbar ist und eine Bestimmung der Größe der Öffnung erlaubt. Dies vereinfacht die Handhabung der Vorrichtung nochmals.

Von Vorteil ist weiterhin, dass konstruktiv aufwendige Führungselemente zur Umwandlung der Aufweitbewegung des Hohlkörpers in eine in Erstreckungsrichtung eines Schafts verlaufende Anzeigebewegung außerhalb des Patientenkörpers nicht erforderlich sind, weil die Größe der Öffnung anhand der Verformung des Hohlkörpers im Röntgen- oder Ultraschallbild gut erkennbar und durch Vergleich mit den Markierungen zuverlässig bestimmbar ist.

Die Expansion oder Aufweitung des Hohlkörpers in radiale Richtung kann einfach erreicht werden, wenn der Hohlkörper aus einem federnden Material gefertigt ist. Es kann auch vorgesehen sein, dass der Hohlkörper aus einem Memory-Metall gefertigt ist. Von Vorteil ist dabei, dass der Hohlkörper aufgrund der Körperwärme des Patienten in Gebrauchsstellung selbsttätig in die expandierte Position wechselt und sich somit selbst auffaltet. Als Memory-Metall ist jedes Metall mit thermisch aktivierbarem Formgedächtnis, beispielsweise Nitinol, verwendbar.

Zweckmäßig ist es dabei, wenn der Hohlkörper im Messbereich mehrere, insbesondere wenigstens zwei, eine zylindrische Mantelfläche beschreibende Einzelstäbe aufweist, welche ausreichend stabil zur Stabilisierung des Hohlkörpers sind und im Berührbereich mit dem Rand der Öffnung weich und nachgiebig sind.

Besonders günstig ist es dabei, wenn die Einzelstäbe in Gebrauchsstellung durch die Öffnung verlaufen und an dem Rand der Öffnung anliegen. Von Vorteil ist dabei, dass die Größe der Öffnung anhand der Verformungen aus der an sich geraden Form der Einzelstäbe heraus beurteilt werden kann.

Gemäß einer Ausgestaltung der Erfindung von eigenständiger Bedeutung kann vorgesehen sein, dass der Hohlkörper miteinander verdrillte Drahtabschnitte aufweist, die in Gebrauchsstellung mit dem Rand der Öffnung in Berührkontakt sind. Die verdrillten Drahtabschnitte stellen eine insbesondere längsgerichtete Stabilisierung des Hohlkörpers bereit, die im Bereich der Öffnung genügend weich und nachgiebig ist, damit die Abmessung des Randes der Öffnung anhand der Verformung der Drahtabschnitte gut im Röntgen- oder Ultraschallbild erkannt werden kann.

Um zu erreichen, dass sich der Hohlkörper selbsttätig an die Öffnung anpasst, kann vorgesehen sein, dass der Hohlkörper an seinen in Gebrauchsstellung beidseits der Öffnung angeordneten Stirnseiten jeweils ein Metalldrahtgeflecht aufweist, welches elastisch gegen eine Rückstellkraft aus einer maximal expandierten Position radial verformbar ist.

Die Rückstellkraft kann durch eine Federkraft der Metalldrähte des Metalldrahtgeflechts aufgebracht sein und/oder es kann vorgesehen sein, dass die stirnseitigen Enden des Hohlkörpers durch ein gegen eine Zugspannung elastisch verformbares und den Hohlkörper vorzugsweise zentral durchlaufendes Verbindungselement verbunden sind. Hierbei sind die Metalldrahtgeflechte so eingerichtet, dass eine Verkürzung des Verbindungselements ein Aufstellen der Metalldrahtgeflechte und damit eine Vergrößerung des Umfangs des Hohlkörpers bewirkt.

Dabei kann vorgesehen sein, dass das Metalldrahtgeflecht eine Federkraft aufbringt, durch welche der Hohlkörper in entspannter Position seinen maximalen Umfang im Bereich der Öffnung annimmt. Diese Federkraft kann auch durch die Körperwärme des Patienten, welche einen Übergang des beispielsweise aus Memory-Metall gefertigten Metalldrahtgeflechts in eine expandierte Form bewirkt, ausgelöst sein.

Um den Hohlkörper durch eine enge Zuführung, beispielsweise ein Blutgefäß, in die Gebrauchsstellung bringen zu können, kann ein Einführkatheder mit einer Aufnahme für den Hohlkörper in radial zusammengefalteter oder komprimierter Form vorgesehen sein. Vorzugsweise ist die Aufnahme hülsenförmig ausgebildet.

Es kann vorgesehen sein, dass der Hohlkörper an einem Zug-und Schubelement befestigt ist und mit dem Zug- und Schubelement in die Aufnahme und aus der Aufnahme bewegbar ist.

Eine einfache Handhabung der Vorrichtung ergibt sich, wenn die Aufnahme an einem den Hohlkörper in Gebrauchsstellung haltenden Schaft des Einführkatheders, insbesondere am distalen Ende, ausgebildet ist.

Gemäß einer Ausgestaltung der Erfindung kann vorgesehen sein, dass die Markierungen an einem den Hohlkörper bei seiner Zuführung und bei seiner Benutzung haltenden Schaft dem Hohlkörper benachbart angeordnet sind. Alternativ oder zusätzlich können die Markierungen an einem mit dem Hohlkörper verbundenen Zug- und Schubelement dem Hohlkörper benachbart angeordnet sein. Von Vorteil ist dabei, dass die Markierungen an einem im Gebrauch nicht oder wenig formveränderlichen Teil in der Nähe der Öffnung angeordnet sind und somit auf dem Röntgen- oder Ultraschallbild der Öffnung mit eingeführtem Höhlkörper zum Größenvergleich gut sichtbar sind.

Es kann auch vorgesehen sein, dass die Markierungen an dem Hohlkörper angeordnet sind. Besonders günstig erweist sich die Anordnung an einem Einzelstab. Hierbei ist eine Änderung des Abstandes der Markierungen voneinander aufgrund der Verformung des Einzelstabes unerheblich, wenn die Öffnung nur in eine Größenklasse eingeordnet werden soll und daher an die Messgenauigkeit geringe Anforderungen gestellt werden.

Die Markierungen können beispielsweise als Verdickungen ausgebildet sein.

Zur freien Auffaltung des Hohlkörpers kann vorgesehen sein, dass der Hohlkörper an einem stirnseitigen Ende auf dem Zug-und Schubelement verschiebbar gelagert ist. Vorzugsweise ist das andere stirnseitige Ende auf dem Zug- und Schubelement fest, also unverschiebbar, angeordnet.

Gemäß einer Ausgestaltung der Erfindung kann vorgesehen sein, dass der Hohlkörper mit dem Schaft und/oder mit dem Zug- und Schubelement lösbar verbunden ist. Von Vorteil ist dabei, dass der Hohlkörper einfach ausgetauscht werden kann. Es können somit auch unterschiedliche Hohlkörper für Messungen in unterschiedlichen Größenbereichen an einer Vorrichtung verwendet werden. Besonders günstig ist es dabei, wenn die Verbindung als Schraubverbindung ausgebildet ist.

Die Erfindung wird nun anhand eines Ausführungsbeispiels näher beschrieben, ist jedoch nicht auf dieses Ausführungsbeispiel beschränkt. Weiter Ausführungsbeispiele ergeben sich durch Kombination der Merkmale der Patentansprüche untereinander und/oder mit einzelnen oder mehreren Merkmalen des Ausführungsbeispiels.

Es zeigt in schematisierter Darstellung
- Fig. 1: eine erfindungsgemäße Vorrichtung in expandierter oder aufgeweiteter Position,
- Fig. 2: die erfindungsgemäße Vorrichtung gemäß Fig. 1 in komprimierter Position und
- Fig. 3: die erfindungsgemäße Vorrichtung gemäß Fig. 1 in Gebrauchsstellung.

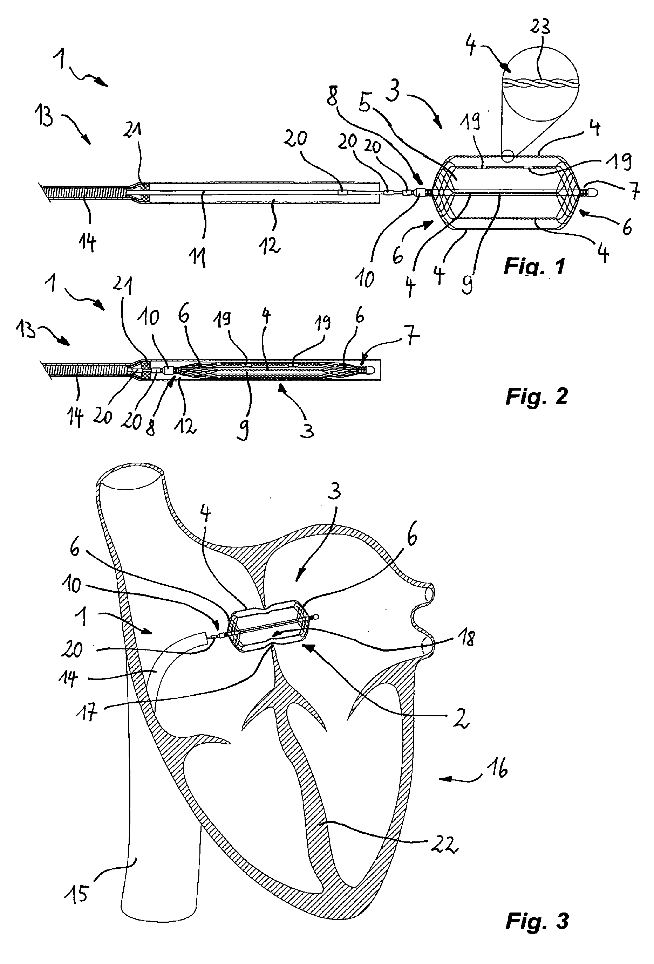

Fig. 1 zeigt teilweise in Schnittdarstellung eine im Ganzen mit 1 bezeichnete Vorrichtung, die zur Messung der Größe einer in Figur 3 ersichtlichen und dort näher beschrieben intrakardialen Öffnung 2 ausgebildete und eingerichtet ist.

Die Vorrichtung 1 weist einen Hohlkörper 3 auf, welcher als Metallkäfig ausgebildet ist. Der Metallkäfig beschreibt somit die durchbrochene Außenfläche des Hohlkörpers 3.

Der Hohlkörper 3 ist aus einem federnden Material hergestellt.

Der Hohlkörper 3 weist mehrere ungefähr oder genau parallel verlaufende Einzelstäbe 4 auf, welche den Hohlkörper 3 in der in Figur 1 gezeigten Position stabilisieren, welche jedoch quer zu ihrer Verlaufsrichtung derart weich ausgebildet sind, dass sie leicht durch Druck von außen in das Innere des Hohlkörpers 3 verformt werden können.

Die Einzelstäbe 4 beschreiben die Mantelfläche einer näherungsweise oder genau zylindrischen Mantelfläche, welche die Wandung 5 des Hohlkörpers 3 bildet.

Die zylinderförmige Mantelfläche der Wandung 5 wird beidseitig begrenzt durch jeweils ein Metalldrahtgeflecht 6. Diese Metalldrahtgeflechte 6 sind jeweils aus miteinander verflochtenen Drähten gebildet, welche zur Bildung der Einzelstäbe 4 im mittleren Bereich des Hohlkörpers 3 verdrillt sind.

Die Einzelstäbe 4, die somit die Unterbrechung des Hohlkörpers 3 zwischen den Metalldrahtgeflechten 6 überbrücken, erlangen durch die Verdrillung eine Längsfestigkeit, durch welche der Hohlkörper 3 in seiner Form stabilisiert wird.

Die Einzelstäbe 4 sind jeweils aus zwei, drei oder mehr parallel geführten und miteinander verdrillten Drähten 23 gebildet. Dadurch, dass diese Drähte 23 einstückig aus den Metalldrahtgeflechten 6 hervorgehen, wird in die Einzelstäbe 4 jeweils eine quer zu deren Erstreckungsrichtung und nach außen gerichtete Federkraft eingebracht.

Jedes Metalldrahtgeflecht 6, ist an einem stirnseitigen Ende 7, 8 des Hohlkörpers 3 mit einem Verbindungselement verbunden.

Das Verbindungselement 9 ist auf Zug längs seiner Erstreckungsrichtung elastisch verformbar und kann daher auf die Metalldrahtgeflechte 6 eine Kraft einbringen, welche gegen die Einzelstäbe 4 wirkt, wodurch ein Auffalten der Metalldrahtgeflechte 6 in die in Fig. 1 gezeigte expandierte oder aufgeweitete Position des Hohlkörpers 3 erreicht wird.

Der Hohlkörper 3 ist über eine Schraubkupplung 10 mit einem Zug- und Schubelement 11 verbunden, mit welchem der Hohlkörper 3 in eine hülsenförmige Aufnahme 12 gebracht werden kann.

Fig. 2 zeigt den Hohlkörper 3 in der Position in der hülsenförmigen Aufnahme 12. Durch die Abmessungen des Innendurchmessers der hülsenförmigen Aufnahme 12 wird hierbei erreicht, dass der Hohlkörper 3 zusammengefaltet und in radialer Richtung komprimiert wird, um in die hülsenförmige Aufnahme 12 zu passen.

Hierbei werden die Metalldrahtgeflechte 6 und gegebenenfalls das Verbindungselement 9 gegen eine Federkraft elastisch verformt, wobei Federkraft die erneute Expansion des Hohlkörpers 3 in die in Fig. 1 gezeigte Position bewirkt, sobald der Hohlkörper 3 mittels des Zug- und Schubelements 11 aus der hülsenförmigen Aufnahme 12 herausgeschoben wird.

Bei einem Ausführungsbeispiel wird dieses Federkraft aufgebaut oder verstärkt, sobald der Hohlkörper 3 in thermischen Kontakt mit einer warmen Umgebung, beispielsweise einem Patientenkörper, kommt, indem der Hohlkörper 3 aus einem Metall mit Formgedächtnis, insbesondere Nitinol, gefertigt wird. Hierbei ist die in dem Formgedächtnis gespeicherte Form des Metallkäfigs des Hohlkörpers 3 die in Fig. 1 gezeigte, expandierte Form.

Bei einem weiteren Ausführungsbeispiel kann vorgesehen sein, dass das Verbindungselement 9 starr ausgeführt ist, wobei das stirnseitige Ende 7 an dem Verbindungselement 9 festgelegt ist, während das stirnseitige Ende 8 auf dem Verbindungselement 9 längs zu dessen Erstreckungsrichtung verschieblich gelagert ist.

Die hülsenförmige Aufnahme 12 ist am distalen Ende 13 eines Schafts 14 eines Venenkatheders angeordnet.

Um die in Figur 3 gezeigte Gebrauchstellung der Vorrichtung 1 zu erreichen, wird das distale Ende 13 mit dem in der Aufnahme 12 angeordneten Hohlkörper 3 durch eine Vene 15 in die intrakardiale Öffnung 2 in einer Scheidewand 22 des Herzens 16 eingeführt. Hierzu werden nicht weiter dargestellte Führungsdrähte oder Führungskatheder verwendet.

Anschließend wird der Schaft 14 zurückgezogen, wobei der Hohlkörper 3 mittels des Zug- und Schubelements 11 aus der Aufnahme 12 herausgeschoben wird und sich in der in Figur 3 dargestellten Weise aufweitet und an den Rand 17 der Öffnung 2 anschmiegt.

Hierbei bewirkt der Rand 17 eine in das Innere des Hohlkörpers 3 gerichtete Verformung der Einzelstäbe 4 im Berührbereich 18. Die dargestellte Verformung ergibt sich auch durch eine nach außen gerichtete Eigenspannung der Einzelstäbe.

Da der Metallkäfig des Hohlkörpers 3 in einer Röntgen- oder Ultraschallaufnahme gut erkennbar ist, kann die Größe der Öffnung 2 aus der Verformung der Einzelstäbe 4 bestimmt werden.

Zum Vergleich von Größenordnungen sind auf den Einzelstäben 4 in Figur 1 ersichtliche Markierungen 19 angebracht, die als Referenzmaßstab verwendet werden können. Da die Größe der Öffnung 2 nur näherungsweise zur Ermittlung eines passenden Verschlusses gemessen werden muss, ist die Änderung des Abstandes der Markierungen 19 voneinander durch die in Figur 3 dargestellte Verformung der Einzelstäbe 4 unerheblich.

Auf dem Zug- und Schubelement 11 sind in einem axialen Abstand zueinander weitere Markierungen 20 als Verdickungen ausgebildet, die im Röntgen- oder Ultraschallbild ebenfalls gut erkennbar sind und auch als Referenzmaßstab für die Größenbestimmung der Öffnung 2 verwendet werden können.

Hierbei sind zwei der Markierungen 20 so angeordnet, dass sie in der Fig. 2 dargestellten, zurückgezogenen Position des Hohlkörpers 3 ein als gelochte Dichtscheibe ausgebildetes Halteelement 21 beidseitig begrenzen und so den Hohlkörper 3 in der Aufnahme 12 gegen ein unbeabsichtigtes Herausrutschen und Aufhalten beispielsweise in der Vene 15 sichern.

Bei der Vorrichtung 1 zur Messung des Durchmessers einer intrakardialen Öffnung 2 ist ein aus einem Metalldrahtgeflecht bestehender Hohlkörper 3 vorgesehen, welcher verdrillte Einzelstäbe aufweist, die den Mantel der durchbrochenen Wandung 5 des Hohlkörpers 3 bilden und welche eine Längsstabilisierung zwischen zwei die Stirnseite des Hohlkörpers 3 bildenden, elastisch verformbaren Metalldrahtgeflechten 6 bilden und in Gebrauchsstellung durch den Rand 17 der Öffnung 2 in einem Berührbereich in das Innere des Hohlkörpers 3 verformbar sind. Mit der Vorrichtung 1 ist somit die Größe der Öffnung 2 bestimmbar, indem in einem Röntgen- oder Ultraschallbild die Verformung der Einzelstäbe 4 durch die Öffnung 2 mit an dem Hohlkörper 3 und/oder an der Vorrichtung 1 dem Hohlkörper 3 benachbart angeordneten Markierungen 19, 20 vergleichbar ist.

## Patentansprüche

1. Vorrichtung (1) zur Messung der Größe einer intrakardialen Öffnung (2), mit einem eine nachgiebige Wandung (5) aufweisenden, in die Öffnung (2) einführbaren und quer zur Einführungsrichtung expandierbaren Hohlkörper (3) und mit wenigstens zwei strahlenundurchlässigen, in einem vorbestimmten Abstand zueinander angeordneten Markierungen (19, 20), deren Abstand mit der Einformung des Hohlkörpers (3) durch den Rand (17)der zu messenden Öffnung (2) verglichen werden kann, wobei der Hohlkörper (3) flüssigkeitsdurchlässig und als ein Metallkäfig ausgebildet ist, **dadurch gekennzeichnet, dass** der Hohlkörper (3) in seinem mittleren Bereich zwischen seinen Stirnseiten (7, 8) unterbrochen und die Unterbrechung durch Einzelstäbe (4) überbrückt ist, welche jeweils durch wenigstens zwei miteinander verdrillte Drähte (23) gebildet sind.

2. Vorrichtung nach Anspruch 1, **dadurch gekennzeichnet, dass** die Einzelstäbe (4) parallel zueinander und zu einer Längsmittelachse des Hohlkörpers (3) angeordnet sind.

3. Vorrichtung (1) nach Anspruch 1 oder 2, **dadurch gekennzeichnet, dass** der Hohlkörper (3) aus einem federnden Material und/oder aus einem Memory-Metall, insbesondere aus Nitinol, gefertigt ist.

4. Vorrichtung (1) nach einem der Ansprüche 1 bis 3, **dadurch gekennzeichnet, dass** der Hohlkörper (3) im Messbereich mehrere eine zylindrische Mantelfläche beschreibende Einzelstäbe (4) aufweist, welche ausreichend stabil zur Stabilisierung des Hohlkörpers (3) sind und im Berührbereich (18) mit dem Rand (17) der Öffnung (2) weich und nachgiebig sind.

5. Vorrichtung (1) nach einem der Ansprüche 1 bis 4, **dadurch gekennzeichnet, dass** die Einzelstäbe (4) in Gebrauchsstellung durch die Öffnung (2) verlaufen und an dem Rand (17) der Öffnung (2) anliegen.

6. Vorrichtung (1) nach einem der Ansprüche 1 bis 5, **dadurch gekennzeichnet, dass** der Hohlkörper (3) miteinander verdrillte Drahtabschnitte (23) aufweist, die in Gebrauchsstellung mit dem Rand (17) der Öffnung (2) in Berührkontakt sind.

7. Vorrichtung nach einem der Ansprüche 1 bis 6, **dadurch gekennzeichnet, dass** der Hohlkörper (3) an seinen in Gebrauchsstellung beidseits der Öffnung (2) angeordneten Stirnseiten (7, 8) jeweils ein Metalldrahtgeflecht (6) aufweist, welches elastisch gegen eine Rückstellkraft aus einer maximal expandierten Position radial verformbar ist.

8. Vorrichtung nach einem der Ansprüche 1 bis 7, **dadurch gekennzeichnet, dass** die stirnseitigen Enden (7, 8) des Hohlkörpers (3) durch ein gegen eine Zugspannung elastisch verformbares und den Hohlkörper (3) vorzugsweise zentral durchlaufendes Verbindungselement (9) verbunden sind.

9. Vorrichtung nach einem der Ansprüche 1 bis 8, **dadurch gekennzeichnet, dass** ein Einführkatheter mit einer hülsenförmigen Aufnahme (12) für den Hohlkörper (3) in radial zusammengefalteter oder komprimierter Form vorgesehen ist.

10. Vorrichtung nach einem der Ansprüche 1 bis 9, **dadurch gekennzeichnet, dass** der Hohlkörper (3) an einem Zug-und Schubelement (11) befestigt ist und mit dem Zug- und Schubelement (11) in die Aufnahme (12) und aus der Aufnahme (12) bewegbar ist.

11. Vorrichtung nach einem der Ansprüche 1 bis 10, **dadurch gekennzeichnet, dass** die Aufnahme (12) an einem den Hohlkörper (3) in Gebrauchsstellung haltenden Schaft (14) des Einführkatheters ausgebildet ist.

12. Vorrichtung nach einem der Ansprüche 1 bis 11, **dadurch gekennzeichnet, dass** die Markierungen (19, 20) an einem den Hohlkörper (3) bei seiner Zuführung und bei seiner Benutzung haltenden Schaft (14) und/oder einem mit dem Hohlkörper (3) verbundenen Zug- und Schubelement (11) dem Hohlkörper (3) benachbart angeordnet sind.

13. Vorrichtung nach einem der Ansprüche 1 bis 12, **dadurch gekennzeichnet, dass** die Markierungen (19, 20) an dem Hohlkörper (3), insbesondere an einem Einzelstab (4), angeordnet sind.

14. Vorrichtung nach einem der Ansprüche 1 bis 13, **dadurch gekennzeichnet, dass** der Hohlkörper (3)an einem stirnseitigen Ende (7, 8) auf einem Zug- und Schubelement (11) verschiebbar gelagert ist.

15. Vorrichtung nach einem der Ansprüche 1 bis 14, **dadurch gekennzeichnet, dass** der Hohlkörper (3) mit einem Schaft (14) und/oder mit einem Zug- und Schubelement (11) lösbar verbunden ist.
